(19) Europäisches Patentamt
European Patent Office
Office européen des brevets

(11) **EP 3 238 629 A1**

(12) **EUROPEAN PATENT APPLICATION**

(43) Date of publication:
01.11.2017 Bulletin 2017/44

(51) Int Cl.:
*A61B 8/00* (2006.01)　　*G01N 29/32* (2006.01)
*B06B 1/02* (2006.01)　　*H04R 19/00* (2006.01)

(21) Application number: 16206869.6

(22) Date of filing: 23.12.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 29.04.2016　IT UA20163033

(71) Applicant: STMicroelectronics S.r.l.
20864 Agrate Brianza (MI) (IT)

(72) Inventors:
• MORELLI, Marco
20010 BAREGGIO (IT)
• QUAGLIA, Fabio
27050 PIZZALE (IT)
• TOIA, Fabrizio Fausto Renzo
21052 BUSTO ARSIZIO (IT)
• SAMBI, Marco
20010 CORNAREDO (IT)
• BARILLARO, Giuseppe
56121 PISA (IT)

(74) Representative: Cerbaro, Elena et al
Studio Torta S.p.A.
Via Viotti, 9
10121 Torino (IT)

(54) **INTEGRATED ACOUSTIC TRANSDUCER WITH REDUCED PROPAGATION OF UNDESIRED ACOUSTIC WAVES**

(57)　The acoustic device has a micromachined acoustic transducer element (15); an acoustically attenuating region (40); and an acoustic matching region (32) arranged between the acoustic transducer element (15) and the acoustically attenuating region (40). The acoustic transducer element (15) is formed in a first substrate (25) housing a cavity (19) delimiting a membrane (16). A second substrate (30) of semiconductor material integrating an electronic circuit is arranged between the acoustic transducer element (15) and the acoustically attenuating region (40). The acoustic matching region (32) has a first interface (32A) with the second substrate (30) and a second interface (32B) with the acoustically attenuating region (40). The acoustic matching region (32) has an impedance matched to the impedance of the second substrate (30) in proximity of the first interface (32A), and an impedance matched to the acoustically attenuating region (40) in proximity of the second interface (32B).

Fig.2

EP 3 238 629 A1

**Description**

The present invention relates to an integrated acoustic transducer with reduced propagation of undesired acoustic waves.

Integrated acoustic transducers made using the semiconductor technology are known, and operate according to a capacitive principle. In some applications, these transducers are used for transducing ultrasonic waves; in this case, they are known as MUTs (Micromachined Ultrasonic Transducers), whether of a capacitive type (CMUTs - Capacitive Micromachined Ultrasonic Transducers) or of a piezoelectric type (PMUTs - Piezoelectric Micromachined Ultrasonic Transducers). For instance, CMUTs are used in ultrasound image generation systems for medical diagnostics.

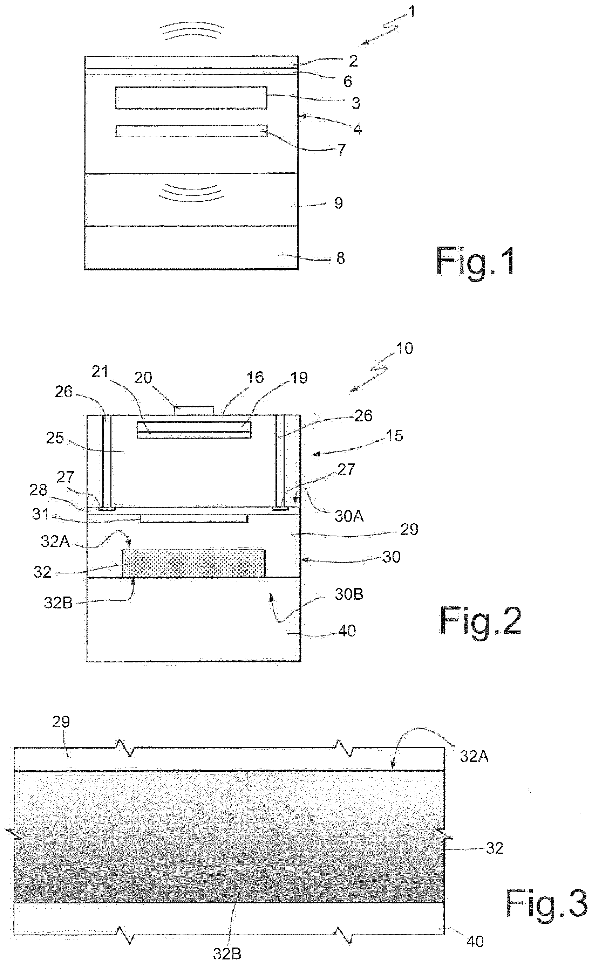

An example of a transducer element of this type is shown in Figure 1.

The transducer element of Figure 1, designated as a whole by reference number 1, comprises a membrane 2, for example, of silicon nitride, suspended over a cavity 3 and formed in or on a silicon chip 4. The cavity 3 may contain air or gas or be partially or totally in vacuum conditions. A conductive material layer, for example of aluminium, is generally formed on the membrane 2 and forms a first electrode 6. Another conductive material layer forms a second electrode 10, within the chip 4, underneath the cavity 3.

Generally, the acoustic transducer element 1 is coupled to a semiconductor material chip integrating an electronic circuit, for example, an ASIC (Application Specific Integrated Circuit) 8, for processing signals generated by or sent to the acoustic transducer element 1. In the embodiment illustrated, the ASIC 8 is fixed on the back of the acoustic transducer element 1. In the transducer element 1 of Figure 1, the first and second electrodes 6, 7 form a capacitor that undergoes a capacitance variation when an acoustic wave hits membrane 2, causing it to deflect. This capacitance variation between the two electrodes 6, 7 may be detected by the electronic circuit, represented integrated in the ASIC 8, thus transducing the acoustic signal into an electrical signal. Likewise, when an a.c. electrical signal is applied to one or both the electrodes 6, 7, it causes a movement of the membrane 2 that consequently generates an acoustic signal. For this reason, the transducer element 1 may operate both as sensor of acoustic waves and as emitter of acoustic waves.

In practical applications, due to the small size of the acoustic transducer elements, of the order of microns, they are generally formed close to one another, so as to form an acoustic device of sizes suited to the envisaged application.

When the acoustic transducer element 1 operates as generator of acoustic waves, it generates the acoustic waves mainly towards the outside world. However, a part of the acoustic energy is propagated back towards the ASIC 8. This acoustic energy may be reflected towards the transducer element 1 because of the interface between the latter and the ASIC 8. To prevent such a back reflection, which could cause undesired interference phenomena with the acoustic signal, it has already been proposed to arrange an attenuating layer 9 between the chip 4 and the ASIC 8 (see, for example, U.S. patents N. 6,831,394 and 7,280,435).

The attenuating layer 9 may for example be formed by a plastic material, such as an epoxy resin, polyvinyl chloride, or Teflon, containing filler material such as silver, tungsten, BN, AIN, or $Al_2O_3$.

The known solutions do not, however, ensure a sufficient reduction of reflection because of the presence of the existing interfaces.

The aim of the present invention is to provide a transducer device that solves the problems encountered in the prior art.

According to the invention, an acoustic transducer device is provided according to the attached claims.

In practice, the present transducer device provides an acoustic matching region arranged between the transducer element and the attenuating layer. The matching region is here of porous silicon and has a variable acoustic impedance throughout its thickness, matched so as to have a value close to that of the adjacent regions. In this way, the acoustic waves that propagate backwards from the membrane do not meet any discontinuity of the acoustic impedance of the traversed media, and reflection of the acoustic waves towards the membrane is reduced.

For a better understanding of the present invention, preferred embodiments thereof are now described purely by way of non-limiting example, with reference to the attached drawings, wherein:

- Figure 1 is a cross-section through a known acoustic transducer element;
- Figure 2 is a cross-section through the present acoustic transducer element;
- Figure 3 shows an enlarged detail of the acoustic transducer element of Figure 2;
- Figure 4 shows an enlarged portion of the detail of Figure 3;
- Figures 5-9 are cross-sections of different embodiments of the present acoustic transducer element; and
- Figure 10 is a cross-section of a device having a plurality of transducer elements shown in Figures 2-9 and formed in a single substrate so as to form an array.

Figure 2 shows an embodiment of an acoustic transducer device, designated as a whole by the reference number 10.

The acoustic transducer device 10 comprises a transducer element 15 formed in a substrate 25 of semiconductor material. The substrate 25 has a cavity 19 that

delims, at the bottom, a membrane 16, a first electrode 20 and a second electrode 21, arranged over the membrane 16 and on the bottom of the cavity 19, respectively. The substrate 15, typically of mono- and/or polycrystalline silicon, may be traversed by through vias 26 of electrically conductive material.

An ASIC 30 is bonded to the substrate 25 on the side thereof remote with respect to the membrane 16. The ASIC 30 has a first face 30A and a second face 30B and comprises a substrate 29 forming an active area 31 facing the first face 30A. The active area 31 accommodates electronic circuits (not illustrated), connected to the substrate 25 of the acoustic transducer element 15 through pads 27 and electrical connection lines (not illustrated). The pads 27 are in contact with the through vias 26 of the substrate 25 of the acoustic transducer element 15, inside an insulating layer 28, overlying the substrate 29.

In Figure 2, the ASIC 30 further forms an acoustic matching element 32, extending from the second face 30B towards the inside of the substrate 29. The acoustic matching element 32 is here in contact with an acoustically attenuating region 40 bonded to the second face 30B of the ASIC 8.

The acoustic matching element 32 forms a first interface 32A with the substrate 29 of the ASIC 30 and a second interface 32B with the acoustically attenuating region 40, as shown in the enlarged detail of Figure 3.

The acoustic matching element 32 is of porous silicon and has a variable impedance between the first and second interfaces 32A, 32B. In detail, the impedance value of the acoustic matching element 32 in proximity of each interface 32A, 32B is chosen to correspond to the acoustic impedance of the material with which it is in contact. In particular, the first interface 32A has an acoustic impedance similar to that of the substrate 29 of the ASIC 30, and the second interface 32B has an acoustic impedance similar to that of the acoustically attenuating region 40.

The impedance matching on the two interfaces 32A, 32B enables a reduction of the reflected acoustic energy. In fact, the acoustic energy reflected on the interface 32A is given by:

$$U_{32A} = \frac{Z_{32A} - Z_{29}}{Z_{32A} + Z_{29}} U_T$$

where $Z_{32A}$ is the impedance of the acoustic matching element 32 in proximity of the first interface 32A, $Z_{29}$ is the impedance of the material of the substrate 29 (silicon), and $U_T$ is the acoustic energy transmitted backwards by the transducer element 15.

By modulating the impedance $Z_{32A}$ of the acoustic matching element 32 in proximity of the first interface 32A so that it is approximately equal to the impedance $Z_{29}$ of the silicon substrate 29, $Z_{32A} \approx Z_{29}$, the reflected acoustic energy may be drastically reduced almost to zero.

Likewise, the acoustic energy reflected on the interface 32B is given by:

$$U_{32B} = \frac{Z_{32B} - Z_{40}}{Z_{32B} + Z_{40}} U_{T1}$$

where $Z_{32B}$ is the impedance of the acoustic matching element 32 in proximity of the second interface 32B, $Z_{40}$ is the impedance of the material of the acoustically attenuating region 40, and $U_{T1}$ is the acoustic energy traversing the second interface 32B.

Also in this case, by modulating the impedance $Z_{32B}$ of the acoustic matching element 32 in proximity of the second interface 32B so that it is approximately equal to the impedance $Z_{40}$ of the acoustically attenuating region 40, $Z_{32A} \approx Z_{40}$, the acoustic energy reflected on the second interface 32B is reduced.

In practice, any acoustic waves that propagate back from the membrane 16 do not encounter any discontinuity in the impedance of the materials that they traverse, and therefore do not generate acoustic waves reflected towards the membrane 16, thus preventing any undesirable interference phenomena with the useful acoustic signal.

Variation of impedance of the acoustic matching element 32 is obtained by modulating the porosity of the porous silicon. In particular, the porosity may be regulated by selectively modifying the size of the pores so that it is smaller in proximity of the first interface 32A and larger in proximity of the second interface 32B, varying continuously from the first interface 32A to the second interface 32B.

The acoustic matching element 32 may, for example, be manufactured by selectively doping the substrate 29 of the ASIC 30 starting from the second face 32A with P-type dopant (for example, boron), and performing an electrochemical etch. In particular, before forming the electrical components in the active part 31, the semiconductor material wafer intended to form the ASIC 30 is implanted with the P-type dopant and then immersed in an acid bath. By applying an appropriate potential difference and modulating the current flowing in the wafer with time, pores are formed within the doped area. In particular, as explained in the article by S. Matthias, F. Müller, J. Schilling, U. Gösele, "Pushing the limits of microporous silicon etching", Appl. Phys. A 80, 1391-1396 (2005), the porosity, and thus the diameter of the pores, as a function of the depth may be modulated by varying the etching parameters, in particular the applied voltage and the current flowing during the etching time so as to obtain the desired impedance values.

The acoustic matching region 32 may also be obtained starting from a region with an N-type doping (for example, doped with phosphorus), which is rendered porous via an electrochemical etch, possibly carried out under exposure to ultraviolet and/or visible light. Also in this case, the porosity, and thus the diameter of the pores, may be modulated as a function of the depth by accordingly varying the etching parameters, in particular the voltage and the current flowing during the etching time.

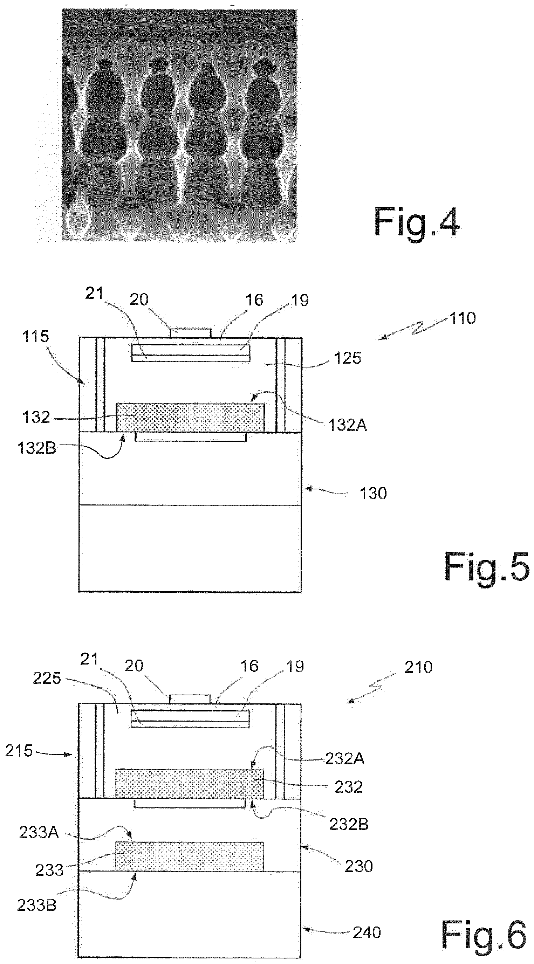

Figure 4 shows in detail an example of the porous silicon structure of Figure 3.

In another embodiment, shown in Figure 5, the acoustic matching element, here designated by 132, is formed within the substrate, here designated by 125, instead of inside the ASIC 130. In this case, the impedance of the interfaces 132A and 132B is similar to that of the substrate 125 and to that of the ASIC 130, respectively.

Figure 6 shows a further embodiment comprising a first and a second acoustic matching element 232, 233. The first acoustic matching element 232 is similar to the acoustic matching element 132 of Figure 5. It is thus formed in the substrate 225 of the acoustic transducer element 215 and has, in proximity of a first interface 232A, an impedance similar to that of the substrate 225, and, in proximity of a second interface 232B, an impedance similar to that of the ASIC 230. The second acoustic matching element 233 is similar to the acoustic matching element 32 of Figure 2. It is thus formed in the ASIC 230 and has, in proximity of a first interface 233A, an impedance similar to that of the ASIC 230, and, in proximity of a second interface 233B, an impedance similar to that of the acoustically attenuating region 240.

In this way, there is a double acoustic matching both between the substrate 225 and the ASIC 230 and between the ASIC 230 and the acoustically attenuating region 240.

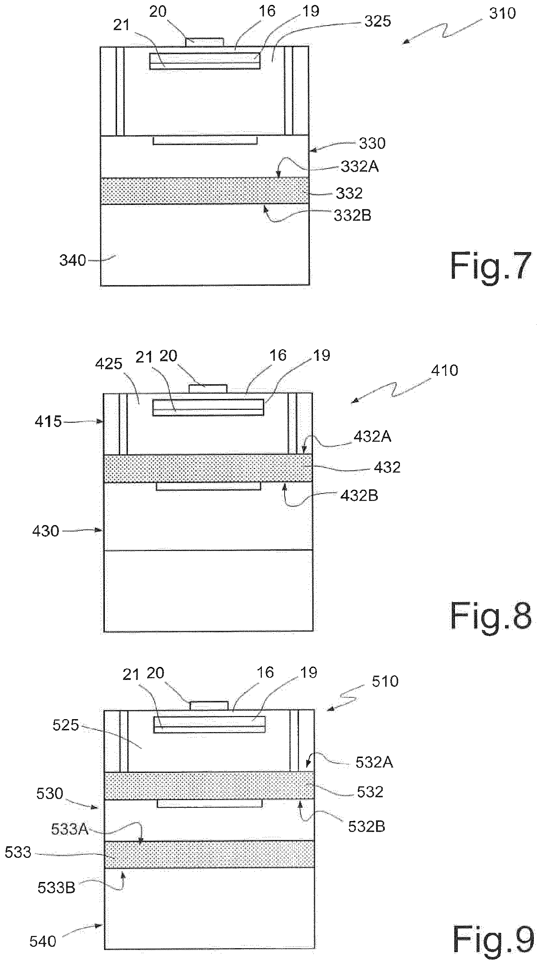

In another embodiment, shown in Figure 7, the acoustic matching element, here designated by 332, is formed as a separate chip, arranged between the ASIC 330 and the acoustically attenuating region 340. Also in this case, the impedance of the faces 332A and 332B is similar to that of the ASIC 330 and to that of the acoustically attenuating region 340, respectively.

In another embodiment, shown in Figure 8, the acoustic matching element, here designated by 432, is formed as a separate chip, arranged between the substrate 425 of the acoustic transducer element 415 and the ASIC 430. Also in this case, the impedance of the faces 432A and 432B is similar to that of the substrate 425 and to that of the ASIC 430, respectively.

Figure 9 shows a variation of the embodiment of Figure 6, wherein the first and second acoustic matching elements, here designated by 532, 533, are both formed in separate dice.

In all the illustrated embodiments, the acoustic matching element or elements 32, 132, 232, 332, 432, 532, 233, 533, reduce generation of undesired reflected waves by eliminating any sharp variations of impedance.

The described solutions further have the advantage that the use of porous silicon enables considerable freedom of design, in particular as regards the reduction of parasitic capacitances between the ASIC 30, 130, 230, 330, 430, 530 and the substrate 25, 125, 225, 325, 425, 525.

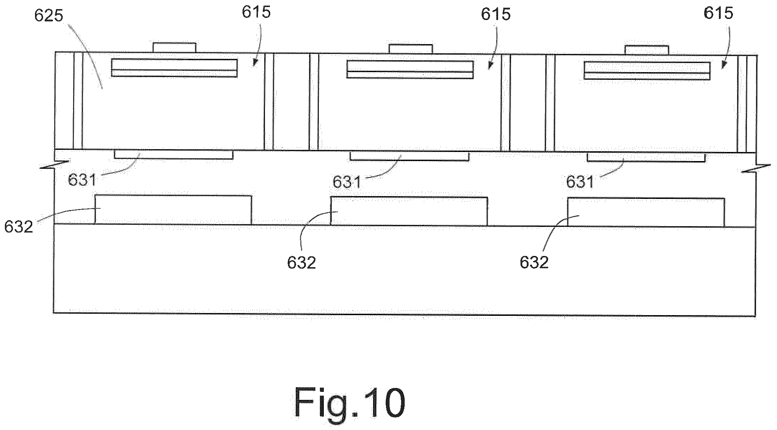

The described acoustic transducer device 10, 110, 210, 310, 410, 510 above may comprise a plurality of transducer elements having the structures illustrated in Figures 2-9 and formed in a single substrate. For instance, Figure 10 shows a substrate 625 housing a plurality of transducer elements 615, each whereof arranged on a respective active area 631 and a respective acoustic matching region 632.

The acoustic transducer device of Figure 10 may form, for example, an ultrasonic transducer (either of a capacitive type, referred to as CMUT, and of a piezoelectric type, referred to as PMUT) for medical use, operating at frequencies comprised between 1 and 15 MHz. It may, however, be used for consumer applications wherein a high degree of miniaturization is desired, such as in gesture recognition mobile devices. Further, it may also be used for high-voltage devices and optical devices.

Finally, it is clear that modifications and variations may be made to the device described and illustrated herein, without thereby departing from the scope of the present invention, as defined in the attached claims.

For instance, the acoustically attenuating region 40 could be arranged between the transducer element 15 and the ASIC 8. In this case, the acoustic matching element may be arranged between the transducer element 15 and the acoustically attenuating region 40.

**Claims**

1. An acoustic device comprising:

   a micromachined acoustic transducer element (15; 115; 215; 315; 415; 515);
   an acoustically attenuating region (40; 140; 240; 340; 340; 440; 540); and
   an acoustic matching region (32; 132; 232; 432; 532; 233; 533) arranged between the acoustic transducer element (15; 115; 215; 315; 415; 515) and the acoustically attenuating region (40; 140; 240; 340; 340; 440; 540).

2. A device according to claim 1, wherein the acoustic transducer element (15; 115; 215; 315; 415; 515) is formed in a first substrate (25, 125; 225; 325; 325; 425; 525) housing a cavity (19) delimiting a membrane (16).

3. A device according to claim 2, further comprising a second substrate (30; 130; 230; 230, 330; 430; 530) of semiconductor material integrating an electronic circuit and arranged between the acoustic transducer element (15; 115; 215; 315; 415; 515) and the acoustically attenuating region (40; 140; 240; 340; 340; 440; 540).

**4.** A device according to claim 3, wherein the acoustic matching region (132; 232; 432; 532) is arranged between the acoustic transducer element (115; 215; 415; 515) and the second substrate (130; 230; 430; 530).

**5.** A device according to claim 4, wherein the acoustic matching region (132; 232) is formed in the first substrate (125, 225) of the acoustic transducer element (115; 215).

**6.** A device according to claim 4, wherein the acoustic matching region (432; 532) is formed in a semiconductor material body arranged between the first substrate (425; 525) and the second substrate (430; 530).

**7.** A device according to claim 5 or 6, wherein the acoustic matching region (232; 532) is a first acoustic matching region, the device further comprising a second acoustic matching region (233; 533) arranged between the second substrate (30) and the acoustically attenuating region (240; 540).

**8.** A device according to claim 7, wherein the second acoustic matching region (233) is formed in the second substrate (230).

**9.** A device according to claim 4, wherein the acoustic matching region (32; 233; 332; 532) is arranged between the second substrate (30; 230; 330; 530) and the acoustically attenuating region (40; 240; 340; 540).

**10.** A device according to claim 9, wherein the acoustic matching region (32; 233) is formed in the second substrate (30; 230).

**11.** A device according to claim 9, wherein the acoustic matching region (332; 533) is formed in a semiconductor material body arranged between the second substrate (330; 530) and the acoustically attenuating region (340; 540).

**12.** A device according to claim 10, wherein the second acoustic matching region (533) is formed in a semiconductor material body arranged between the second substrate (530) and the acoustically attenuating region (540).

**13.** A device according to any of claims 3-12, wherein the first acoustic matching element (32; 132; 232; 432; 532; 233; 533) comprises a variable impedance layer.

**14.** A device according to claim 13, wherein the acoustic matching region (32; 132; 232; 432; 532; 233; 533) has a first interface (32A; 132A; 232A; 432A; 532A; 233A; 533A) with a first element chosen between the acoustic transducer element (15; 115; 215; 315; 415; 515) and the second substrate (30; 130; 230; 230, 330; 430; 530) and a second interface (32B; 132B; 232B; 432B; 532B; 233B; 533B) with a second element chosen between the second substrate (30; 130; 230; 230, 330; 430; 530) and the acoustically attenuating region (40; 140; 240; 340; 340; 440; 540), the first element having a first impedance and the second element having a second impedance, and the acoustic matching region (32; 132; 232; 432; 532; 233; 533) has a third impedance in proximity of the first interface, matched to the first impedance, and a fourth impedance in proximity of the second interface, matched to the second impedance.

**15.** A device according to any of claims 1-14, wherein the acoustic matching region (32; 132; 232; 432; 532; 233; 533) is of porous silicon.

**16.** A device according to claim 15 when depending on claim 14, wherein the acoustic matching region (32; 132; 232; 432; 532; 233; 533) has a plurality of pores, wherein the sizes of the pores are variable between the first and second interfaces (32A; 132A; 232A; 432A; 532A; 233A; 533A, 32B; 132B; 232B; 432B; 532B; 233B; 533B).

**17.** A device according to any of the preceding claims, forming an ultrasonic transducer (CMUT; PMUT) for medical use.

Fig.1

Fig.2

Fig.3

Fig.4

Fig.5

Fig.6

Fig.7

Fig.8

Fig.9

Fig.10

EP 3 238 629 A1

Europäisches
Patentamt

European
Patent Office

Office européen
des brevets

# EUROPEAN SEARCH REPORT

Application Number

EP 16 20 6869

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 154 057 B2 (AKIYAMA TAKAHIRO [JP] ET AL) 6 October 2015 (2015-10-06) | 1-14,17 | INV.<br>A61B8/00 |
| Y | * figures 1,3 *<br>* column 3, line 54 - column 6, line 35 *<br>* page 7, line 50 - page 8, line 10 *<br>----- | 15,16 | G01N29/32<br><br>ADD.<br>B06B1/02 |
| Y | US 2007/164632 A1 (ADACHI HIDEO [JP] ET AL) 19 July 2007 (2007-07-19) | 15,16 | H04R19/00 |
| A | * figure 30 *<br>* paragraph [0342] - paragraph [0346] *<br>----- | 1-14,17 | |

TECHNICAL FIELDS
SEARCHED (IPC)

A61B
B06B
G01N
H04R

The present search report has been drawn up for all claims

| Place of search | Date of completion of the search | Examiner |
|---|---|---|
| Munich | 3 July 2017 | Moscu, Viorel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

                                                   
& : member of the same patent family, corresponding
document

EPO FORM 1503 03.82 (P04C01)

# EP 3 238 629 A1

## ANNEX TO THE EUROPEAN SEARCH REPORT
## ON EUROPEAN PATENT APPLICATION NO.

EP 16 20 6869

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2017

| Patent document cited in search report | | Publication date | Patent family member(s) | | Publication date |
|---|---|---|---|---|---|
| US 9154057 | B2 | 06-10-2015 | CN | 102933319 A | 13-02-2013 |
| | | | CN | 104815790 A | 05-08-2015 |
| | | | EP | 2576084 A1 | 10-04-2013 |
| | | | JP | 5570311 B2 | 13-08-2014 |
| | | | JP | 2011259094 A | 22-12-2011 |
| | | | US | 2013081471 A1 | 04-04-2013 |
| | | | US | 2015372618 A1 | 24-12-2015 |
| | | | WO | 2011155163 A1 | 15-12-2011 |
| US 2007164632 | A1 | 19-07-2007 | EP | 1761104 A1 | 07-03-2007 |
| | | | JP | 4347885 B2 | 21-10-2009 |
| | | | JP | 4981847 B2 | 25-07-2012 |
| | | | JP | 2009194934 A | 27-08-2009 |
| | | | JP | WO2005120130 A1 | 03-04-2008 |
| | | | US | 2007164632 A1 | 19-07-2007 |
| | | | US | 2008139946 A1 | 12-06-2008 |
| | | | WO | 2005120130 A1 | 15-12-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6831394 B

- US 7280435 B

**Non-patent literature cited in the description**

- **S. MATTHIAS ; F. MÜLLER ; J. SCHILLING ; U. GÖSELE.** Pushing the limits of microporous silicon etching. *Appl. Phys. A,* 2005, vol. 80, 1391-1396